(19) Europäisches Patentamt
European Patent Office
Office européen des brevets

(11) Veröffentlichungsnummer: **0 014 929**
**B1**

(12) # EUROPÄISCHE PATENTSCHRIFT

(45) Veröffentlichungstag der Patentschrift: **06.01.82**

(51) Int. Cl.³: **C 12 Q 1/00**, C 12 Q 1/34

(21) Anmeldenummer: **80100671.9**

(22) Anmeldetag: **11.02.80**

(54) **Diagnostisches Mittel, Verfahren zu dessen Herstellung sowie dessen Verwendung zum Nachweis von Leukozyten in Körperflüssigkeiten.**

(30) Priorität: **14.02.79 DE 2905531**

(43) Veröffentlichungstag der Anmeldung:
**03.09.80 Patentblatt 80/18**

(45) Bekanntmachung des Hinweises auf die Patenterteilung:
**06.01.82 Patentblatt 82/1**

(84) Benannte Vertragsstaaten:
**AT BE CH DE FR GB IT LU NL SE**

(56) Entgegenhaltungen:
**DE-A-2 826 965**
**GB-A-1 128 371**
**US-A-3 290 117**
**US-A-3 853 472**
**US-A-3 874 852**
**US-A-3 917 452**
**CHEMICAL ABSTRACTS, Band 76, Nr. 5, 31. Januar 1972, Zusammenfassung Nr. 22256j, Seite 128, Columbus, Ohio, US, J. RUDENS et al.: »Cytochemical detection of naphthol AS-D chloroacetate esterase«**
**CHEMICAL ABSTRACTS, Band 29, Nr. 5, 31. Juli 1978, Zusammenfassung Nr. 38754z, Seite 239, Columbus, Ohio, US,**
**CHEMICAL ABSTRACTS, Band 89, Nr. 1, 3. Juli 1978, Zusammenfassung Nr. 2110x, Seite 200, Columbus, Ohio, US, G. W. OSBALDISTON et al.: »Cytochemical demonstration of esterases in peripheral blood leukocytes«**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Berger, Dieter, Dr.rer.nat., Bensheimer-Strasse 45, D-6806 Viernheim (DE)**
Erfinder: **Frey, Günter, Pfalzgrafenstrasse 7, D-6701 Ellerstadt (DE)**
Erfinder: **Kuhr, Manfred, Dr.rer.nat., Werthmannweg 23, D-6800 Mannheim 31 (DE)**
Erfinder: **Werner, Wolfgang, Dr.rer.nat., Meissner Weg 39, D-6800 Mannheim 42 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 014 929 B1

Diagnostisches Mittel, Verfahren zu dessen Herstellung sowie dessen Verwendung zum Nachweis von Leukozyten in Körperflüssigkeiten

Der Nachweis von Leukozyten in Körperflüssigkeiten, insbesondere im Urin, nimmt eine hervorragende Stelle in der Diagnostik der Erkrankungen der Niere und des Urogenitaltraktes ein.

Bisher wurde dieser Nachweis geführt durch mikroskopisches Auszählen der Leukozyten im nicht zentrifugierten Harn oder im Harnsediment.

Beiden Methoden ist naturgemäß gemeinsam, daß nur intakte Leukozyten erfaßt werden. Andererseits ist bekannt, daß die Geschwindigkeit der Leukozyten-Lyse je nach Harnmilieu enormen Schwankungen unterworfen ist; so ist z. B. in stark alkalischen Harnen mit einer Leukozyten-Halbwertszeit von nur 60 Minuten zu rechnen. Zu niedrige Leukozytenzahlen bzw. bei längeren Harnstandzeiten sogar falschnegative Befunde sind die Folge.

Vom Lyse-Fehler abgesehen, liefert die quantitative mikroskopische Bestimmung der Leukozyten im nicht zentrifugierten, homogenisierten Harn in der Zählkammer recht zuverlässige Werte. In der Praxis wird diese Methode jedoch nur selten angewandt, da sie mühevoll, ermüdend und zeitraubend ist und den Einsatz geschulten Personals bedingt.

Die überwiegende Mehrzahl der Leukozytenbestimmungen im Harn werden in der medizinischen Praxis nach der sogenannten 'Gesichtsfeldmethode im Harnsediment durchgeführt. Hierzu muß zunächst das Untersuchungsgut (Sediment) durch Zentrifugieren gewonnen werden. Dabei werden jedoch auch andere Bestandteile des Harnes angereichert, die — wie z. B. Salze und Epithelzellen — die mikroskopische Auszählung der Leukozyten beträchtlich erschweren können. Schwankender Sedimentgehalt, Inhomogenitäten des Sedimentes, sowie womöglich unterschiedliche mikroskopische Vergrößerungen oder unterschiedliche optische Ausstattung der Mikroskope führen dazu, daß die hier übliche Angabe über die Anzahl der Leukozyten pro mikroskopischem Gesichtsfeld mit Fehlern von mehreren hundert Prozent behaftet sein kann.

In jüngster Zeit hat es daher nicht an Versuchen gefehlt, ein diagnostisches Mittel bereitzustellen, mit dem die Leukozyten in Körperflüssigkeiten auf einfache und leicht zu handhabende Weise sowie möglichst schnell und vollständig nachgewiesen werden können. Als Nachweisprinzip für einen solchen Leukozytentest haben sich enzymatische Reaktionen angeboten, da die Leukozyten ein breitgefächertes Enzymspektrum besitzen.

In den europäischen Patentanmeldungen EP-A-0 007 407, EP-A-0 008 428 und EP-A-0 012 957 sind beispielsweise diagnostische Mittel beschrieben, die aus einem saugfähigen Träger bestehen, der mit einer geeigneten Puffersubstanz, üblichen Hilfsstoffen und einem Chromogen imprägniert ist. Als Chromogene werden dabei gemäß Patentanmeldung EP-A-0 007 407 Sulfonphthalein-Ester der allgemeinen Formel A

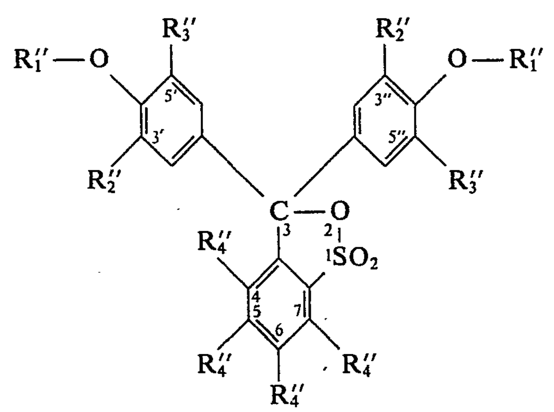

(A)

in der

$R_1''$ einen gegebenenfalls durch Halogen oder eine niedere Alkoxygruppe substituierten Carbonsäurerest, einen mit einer in der Peptidchemie üblichen Stickstoffschutzgruppe versehenen Aminosäure- oder Peptidrest,

$R_2''$ ein Halogenatom oder eine niedere Alkylgruppe,

$R_3''$ und $R_4''$, die gleich oder verschieden sein können, jeweils ein Wasserstoff- oder ein Halogenatom bedeuten,

gemäß Patentanmeldung EP-A-0 008 428 Azofarbstoff-Ester der allgemeinen Formel B

$$A'-N{=}N-B'(OR)_n \qquad (B)$$

in der

A' einen fünf- oder sechsgliedrigen, gegebenenfalls benzoannellierten Rest mit ein bis zwei Heteroatomen aus der Gruppe N, S, O, der gegebenenfalls ein- oder mehrfach durch Halogen,

niedere Alkyl- und/oder niedere Alkoxygruppen substituiert sein kann oder einen ein- bis dreifach durch niedere Alkyl-, niedere Alkoxy-, Nitro-, Sulfonato- und/oder Acylaminogruppen substituierten Phenylrest,

B′ einen gegebenenfalls ein- bis zweifach durch Sulfonato-, niedere Alkoxy- und/oder niedere Alkoxy-poly-alkylenoxy-Gruppen substituierten Benzol-, Naphthalin- oder Chinolin-Rest,

R einen Carbonsäurerest oder einen mit einer in der Peptidchemie üblichen Stickstoffschutzgruppe versehenen Aminosäure- oder Peptid-Rest und

n die Zahl 1 oder 2 bedeuten,

und gemäß Patentanmeldung EP-A-0 012 957 Indoxyl-Ester der allgemeinen Formel C

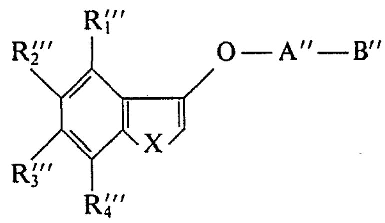

(C)

in der

$R_1'''$, $R_2'''$, $R_3'''$, $R_4'''$, die gleich oder verschieden sein können, jeweils Wasserstoff oder Halogen, eine niedere Alkyl-, niedere Alkoxy-, Aryl-, Aralkyl-, Aralkoxy-, eine Hydroxy-, eine Carboxy-, eine Carboxy-nieder-Alkoxy-, eine Aralkoxycarbonyl-, eine Aralkoxycarbonyl-nieder-Alkoxy-, eine Nitro- oder eine niedere Acylaminogruppe oder jeweils zwei benachbarte Substituenten einen gegebenenfalls durch Halogen substituierten benzoannellierten Rest,

X ein Schwefelatom oder eine gegebenenfalls durch einen niederen Alkyl-, einen Aryl-, einen Aralkyl- oder einen Acylrest substituierte Iminogruppe,

A″ einen Aminosäure- oder einen Peptidrest,

B″ eine in der Peptidchemie übliche oder davon abgeleitete Stickstoffschutzgruppe bedeutet,

eingesetzt.

Mit Hilfe dieser diagnostischen Mittel kann der Nachweis esterolytischer bzw. proteolytischer Enzyme, insbesondere der in den Leukozyten vorhandenen Esterasen bzw. Proteasen in Körperflüssigkeiten, insbesondere im Harn, einfach und rasch über einen Farbumschlag geführt werden.

Es war nun Aufgabe der vorliegenden Erfindung, nach Möglichkeiten zu suchen, die diesem enzymatischen Test zugrunde liegende Nachweisreaktion zu beschleunigen.

Überraschenderweise wurde gefunden, daß die Reaktionszeiten dieser enzymatisch geführten Leukozytenteste erheblich verkürzt werden können, wenn man zusätzlich zu den bisher üblichen Hilfsmitteln und Chromogenen ein oder mehrere Aktivatoren einsetzt.

Gegenstand der vorliegenden Erfindung ist daher ein diagnostisches Mittel zum Nachweis esterolytischer und/oder proteolytischer Enzyme enthaltend neben üblichen Hilfsstoffen ein übliches Esterase- und/oder Proteasesubstrat, dadurch gekennzeichnet, daß es zusätzlich ein oder mehrere Aktivatoren enthält, ausgewählt aus der Gruppe der

a) Pyridin-Derivate der allgemeinen Formel I

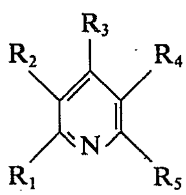

(I)

in der

$R_1$, $R_2$, $R_3$, $R_4$ und $R_5$, die gleich oder verschieden sein können, jeweils Wasserstoff oder Halogen, eine niedere Alkyl- oder eine niedere Alkoxygruppe oder eine Vinylgruppe, die durch einen gegebenenfalls eine oder mehrere niedere Alkoxy-, Amino-, Alkylamino-, Dialkylaminogruppen tragenden Arylrest oder durch einen heterocyclischen Rest substituiert ist, bedeuten,

jeweils zwei benachbarte Substituenten einen gegebenenfalls ein- oder mehrfach durch Halogen, Hydroxygruppe, niedere Alkyl- oder niedere Alkoxygruppen substituierten indeno- oder benzoanellierten Rest, der wiederum einen gegebenenfalls durch eine niedere Alkylgruppe substituierten benzo- oder pyridoanellierten Rest tragen kann, vorstellen können

und $R_3$ zusätzlich für eine Vinyl-chinuclidyl-Carbinol-Gruppe stehen kann.

b) Imidazol-Derivate der allgemeinen Formel II

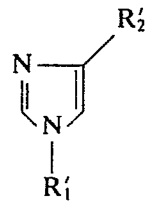

(II)

in der

$R_1'$ Wasserstoff, eine niedere Alkylgruppe oder einen gegebenenfalls durch eine Hydroxy- oder eine Acylgruppe substituierten Arylrest und

$R_2'$ Wasserstoff, eine Aminoalkyl-, N-Acylaminoalkyl- oder einen niederen aliphatischen, gegebenenfalls ungesättigten Carbonsäure-Rest oder einen gegebenenfalls am Stickstoff acylierten niedereren aliphatischen α-Aminosäure-Rest,

bedeuten.

c) Alkohole der allgemeinen Formel III

$$X — A — OH \qquad (III)$$

in der

X Wasserstoff oder eine Hydroxygruppe und

A einen Kohlenwasserstoffrest

bedeuten.

d) Metallkomplexe der allgemeinen Formel IV

$$D_m[B(CN)_n(NO)_p] \qquad (IV)$$

in der

D ein Alkalimetallion,

B ein Schwermetallion,

m eihe ganze Zahl von 2 bis 5,

n eine ganze Zahl von 4 bis 8 und

p 0 oder 1

bedeuten, wobei sich die Zahl m aus der Wertigkeit des Schwermetallions und der Zahl n ergibt.

Ein weiterer Gegenstand der vorliegenden Anmeldung sind Verfahren zur Herstellung dieser diagnostischen Mittel sowie deren Verwendung zum Nachweis von Leukozyten in Körperflüssigkeiten, insbesondere im Harn.

Das erfindungsgemäße diagnostische Mittel besteht vorzugsweise aus einem saugfähigen Träger, einer Filmschicht, einer Pulvermischung, einem Lyophilisat, einer Lösung oder einer Reagenztablette, die ein übliches Esterase- und/oder Proteasesubstrat, jeweils übliche Zusatzstoffe sowie ein oder mehrere der oben erwähnten Aktivatoren enthalten.

Unter Halogen in der Definition der Substituenten $R_1$, $R_2$, $R_3$, $R_4$ und $R_5$ ist Fluor, Chlor, Brom und Jod, vorzugsweise Chlor und Brom zu verstehen.

Die niederen Alkyl- und Alkoxygruppen in der Definition der Substituenten $R_1$, $R_2$, $R_3$, $R_4$ und $R_5$ sowie die niederen Alkylgruppen in der Definition der Substituenten $R_1'$ und $R_2'$ können geradkettig oder verzweigt sein und 1 bis 5, vorzugsweise 1 bis 3 Kohlenstoffatome enthalten. Besonders bevorzugt sind die Methyl-, Ethyl-, n-Propyl- und Isopropyl- bzw. die Methoxy- und Ethoxygruppe.

Der Kohlenwasserstoffrest A kann geradkettig oder verzweigt, gesättigt oder ungesättigt, cyclisch oder acyclisch sein und enthält 1 bis 30, vorzugsweise 5 bis 22 Kohlenstoffatome bei den acyclischen Verbindungen und 3 bis 20, vorzugsweise 6 bis 17 Kohlenstoffatome bei den cyclischen Verbindungen.

Als Acylreste in der Definition der Substituenten $R_1'$ und $R_2'$ kommen die Reste aliphatischer Carbonsäuren mit 1 bis 5, vorzugsweise 1 bis 3 Kohlenstoffatomen in Frage, wobei der Acetylrest ganz besonders bevorzugt ist.

Unter einem Arylrest in der Definition der Substituenten $R_1$, $R_2$, $R_3$, $R_4$, $R_5$ und $R_1'$ sind vorzugsweise die Phenyl- oder die Naphthylgruppe zu verstehen. Als Substituent $R_1'$ ist der Phenylrest ganz besonders bevorzugt.

Unter einem heterocyclischen Rest in der Definition der Substituenten $R_1$, $R_2$, $R_3$, $R_4$ und $R_5$ sind fünf- oder sechsgliedrige Reste mit 1 bis 3 Heteroatomen zu verstehen, wobei als Heteroatome Stickstoff-, Schwefel- und Sauerstoffatome in Frage kommen. Besonders bevorzugt sind der Pyridyl-, Furyl- und Thienyl-Rest.

Als »niederer aliphatischer, gegenenfalls ungesättigter Carbonsäure-Rest« und »gegebenenfalls am Stickstoff acylierter niederer aliphatischer α-Aminosäure-Rest« $R_2'$ kommen Carbonsäuren mit 1 bis 5 vorzugsweise 1 bis 3 Kohlenstoffatomen bzw. deren α-Amino-Derivate in Frage. Besonders bevorzugt sind Essigsäure, Propionsäure und Acrylsäure bzw. L-Alanin und N-Acetyl-L-alanin.

In den Aktivatoren der allgemeinen Formel IV kommen als Alkalimetallionen D vorzugsweise Natrium- und Kalium-Ionen und als Schwermetallionen B vorzugsweise Ionen der Metalle Eisen, Nickel, Chrom, Mangan, Kobalt, Molybdän und Vanadium in Frage.

Als im Sinne der vorliegenden Erfindung einsetzbare Aktivatoren seien beispielhaft erwähnt:

1. Pyridin
2. 2-Methyl-pyridin
3. 3-Äthyl-pyridin
4. 2-Brom-pyridin
5. 3,5-Dichlor-pyridin
6. 4-Methoxy-pyridin
7. 2,6-Dimethyl-4-äthoxy-pyridin
8. Chinolin
9. 2-Methyl-chinolin
10. 8-Methyl-chinolin
11. 7-Isopropyl-chinolin
12. 2-Chlor-chinolin
13. 4-Brom-chinolin
14. 3-Methoxy-chinolin
15. 6-Äthoxy-chinolin
16. 2-Methyl-6-brom-chinolin
17. 2-Methyl-4-methoxy-chinolin
18. 5,7-Dibrom-8-methoxy-chinolin
19. Isochinolin
20. 1-Methyl-isochinolin
21. 3-Propyl-isochinolin
22. 7.-Methyl-isochinolin
23. 1-Chlor-isochinolin
24. 4-Brom-isochinolin
25. 7-Methoxy-isochinolin
26. 1-Methoxy-3-chlor-isochinolin
27. 1-Chlor-4-methyl-5-methoxy-isochinolin
28. Benzo-[b]-chinolin (=Acridin)
29. Benzo-[c]-chinolin (=Phenanthridin)
30. 2-Methyl-phenanthridin
31. 2-Äthyl-phenanthridin
32. 2-Propyl-phenanthridin
33. 2-Methoxy-phenanthridin
34. Benzo-[f]-chinolin
35. 2-Isopropyl-benzo-[f]-chinolin
36. 3-Methyl-benzo-[f]-chinolin
37. 2,4-Dimethyl-benzo-[f]-chinolin
38. Benzo-[g]-chinolin
39. 4-Methyl-benzo-[g]-chinolin
40. 2,4-Dimethyl-benzo-[g]-chinolin
41. Benzo-[h]-chinolin
42. 1,7-Phenanthrolin
43. 2-Methyl-1,7-Phenanthrolin
44. 2,8-Dimethyl-1,7-Phenanthrolin
45. 4,7-Phenanthrolin
46. 3-Methyl-4,7-Phenanthrolin
47. 3,8-Dimethyl-4,7-Phenanthrolin
48. 1,10-Phenanthrolin
49. 2,9-Dimethyl-1,10-Phenanthrolin
50. 4-Aza-fluoren
51. Chinin
52. Chinidin
53. Cinchonin
54. Cinchonidin
55. Cuprein
56. 2-[Phenyl]-vinyl-pyridin-(2′)
57. 2-[4″-Methoxy-phenyl]-vinyl-pyridin-(2′)
58. 2-[4″-(N,N-Dimethylamino)-phenyl]-vinyl-pyridin-(2′)
59. Bis-[2-(Phenyl)-vinyl]-pyridin-(2′,4′)
60. 2-[Naphthyl-(1″)]-vinyl-pyridin-(2′)

61. 2-[Pyridyl-(2″)]-vinyl-pyridin-(2′)
62. 2-[Pyridyl-(3″)]-vinyl-pyridin-(2′)
63. 2-[Pyridyl-(4″)]-vinyl-pyridin-(2′)
64. 2-[Furyl-(2″)]-vinyl-pyridin-(2′)
65. 2-[Pyridyl-(3″)]-vinyl-pyridin-(3′)
66. 2-[Pyridyl-(3″)]-vinyl-pyridin-(4′)
67. 2-[Pyridyl-(4″)]-vinyl-pyridin-(4′)
68. 2-[Thienyl-(2″)]-vinyl-pyridin-(4′)
69. Imidazol
70. 1-Äthyl-imidazol
71. 1-Phenyl-imidazol
72. 1-(4′-Hydroxy-phenyl)-imidazol
73. 1-(4′-Acetyl-phenyl)-imidazol
74. Histamin
75. N-α-Acetyl-histamin
76. (Imidazolyl-4)-essigsäure
77. β-(Imidazolyl-4)-propionsäure
78. β-(Imidazolyl-4)-acrylsäure
79. L-Histidin
80. N-α-Acetyl-L-histidin
81. Hexanol-(1)
82. Heptanol-(1)
83. Octanol-(1)
84. Nonanol-(1)
85. Decanol-(1)
86. Dodecanol-(1)
87. Tetradecanol-(1)
88. Pentadecanol-(1)
89. Hexadecanol-(1)
90. Heptadecanol-(1)
91. Octadecanol-(1)
92. Nonadecanol-(1)
93. Eicosanol-(1)
94. Docosanol-(1)
95. Cyclohexanol
96. Cyclohexen-(1)-ol-(1)
97. Cycloheptanol
98. Cyclooctanol
99. Cyclononanol
100. Cyclodecanol
101. Cyclododecanol
102. Cycloheptadecanol
103. Cycloheptadecen-(9)-ol-(1)
104. Citronellol
105. Geraniol
106. Nerol
107. Linalool
108. Farnesol
109. Nerolidol
110. cis-Octadecen-(9)-ol-(1)
111. Phytol
112. Pentandiol-(1,5)
113. Hexandiol-(1,6)
114. Heptandiol-(1,7)
115. Octandiol-(1,8)
116. Nonandiol-(1,9)
117. Decandiol-(1,10)
118. Dodecandiol-(1,12)
119. Tri-kalium-hexacyano-ferrat-III
120. Tetra-kalium-hexacyano-ferrat-II
121. Di-kalium-tetracyano-nickelat-II
122. Tri-natrium-octacyano-molybdat-V
123. Di-natrium-pentacyano-nitrosyl-ferrat-II
124. Tri-kalium-pentacyano-nitrosyl-manganat-I
125. Tri-kalium-pentacyano-nitrosyl-chromat-I

126. Tri-kalium-pentacyano-nitrosyl-kobaltat-I
127. Penta-kalium-pentacyano-nitrosyl-vanadat-I

Sämtliche Aktivatoren sind bekannte Verbindungen oder können in Analogie zu bekannten Verbindungen hergestellt werden.

Aus den US-Patenten 3 853 472 und 3 917 452 ist bekannt, daß Chinolin-Verbindungen bzw. Vinyl-Pyridin-Verbindungen die peroxidatischen Prozesse beim kolorimetrischen Nachweis von Blut und anderen peroxidatisch wirksamen Substanzen mit Hilfe geeigneter chromogener Substrate und Wasserstoffperoxid zu beschleunigen vermögen. Im Sinne der vorliegenden Erfindung wirken diese Substanzen als Aktivatoren beim Nachweis von Leukozytenesterasen und/oder -proteasen, d. h. bei völlig anderen empfindlicheren und komplexeren Enzymsystemen, bei denen keinerlei peroxidatische Prozesse stattfinden.

Die erfindungsgemäß als Aktivatoren verwendeten Verbindungen der allgemeinen Formeln I, II und IV werden in Konzentrationen von $10^{-4}$ bis 1 mol/Liter, vorzugsweise $10^{-3}$ bis $10^{-1}$ mol/Liter Imprägnierlösung verwendet, die Aktivatoren der allgemeinen Formel III werden der Imprägnierlösung in 0,5—10% (w/v), vorzugsweise 1—5% (w/v) zugesetzt.

Die erfindungsgemäßen diagnostischen Mittel enthalten neben den Aktivatoren die weiteren, üblicherweise verwendeten Bestandteile, wie z. B. übliche Esterase- oder Protease-Substrate, Puffer, Netzmittel, gegebenenfalls Komplexbildner und Oxidationsmittel. Diese werden in der Art und Weise und in den Konzentrationen eingesetzt, wie sie in den EP-A-0 007 407, EP-A-0 008 428 und EP-A-0 012 957 beschrieben sind.

So werden die als Esterase- oder Protease Substrate verwendeten Chromogene üblicherweise in Konzentrationen von $10^{-4}$ bis 1 mol/Liter, vorzugsweise $10^{-3}$ bis $10^{-1}$ mol/Liter Imprägnierlösung, Beschichtungsmasse oder zu untersuchender Flüssigkeit eingesetzt.

Ein weiterer Bestandteil des diagnostischen Mittels zum Nachweis esterolytischer und/oder proteolytischer Enzyme, insbesondere der Leukozyten-Proteasen, ist ein geeignetes Puffersystem. Hierzu kommen z. B. Phosphat-, Borat-, Barbiturat-, Tris-(hydroxymethyl)-aminomethan-(=Tris-)-, 2-Amino-2-methylpropandiol-1,3-(=Amediol)- oder Aminosäure-Puffer in Frage, wobei pH-Wert und Kapazität so gewählt werden müssen, daß sich in der Meßlösung bzw. auf dem Teststreifen ein pH-Wert von 6—10, vorzugsweise von 7—9, einstellt.

Ein weiterer Bestandteil eines diagnostischen Mittels für den Nachweis esterolytischer und/oder proteolytischer Enzyme, insbesondere der Leukozytenesterasen- oder Proteasen, kann ein Netzmittel sein. Vorzugsweise werden nichtionogene aber auch amphotere, kationen- oder anionenaktive Netzmittel in Konzentrationen von 0.05%, vorzugsweise 0.1—1%, eingesetzt.

Ein weiterer Bestandteil des erfindungsgemäßen diagnostischen Mittels kann ein geeigneter Komplexbildner sein. Vorzugsweise werden Metallsalze, beispielsweise der Elemente Eisen, Kupfer, Chrom, Kobalt, Nickel, Mangan und Zink, verwendet. Sie werden in Konzentrationen von $10^{-4}$ bis $10^{-1}$ mol/Liter, vorzugsweise $10^{-3}$ bis $10^{-2}$ mol/Liter Imprägnierlösung eingesetzt.

Weiterhin kann man bei der Herstellung des erfindungsgemäßen diagnostischen Mittels zusätzlich Oxidationsmittel verwenden, wie z. B. Kalium-hexacyanoferrat-III, Kaliumbromat, Kaliumchromat, Phenazin-methosulfat oder Tetrazolium-salze. Diese werden in Konzentrationen von $10^{-4}$ bis 1 mol/Liter, vorzugsweise $10^{-3}$ bis $10^{-1}$ mol/Liter Imprägnierlösung, Beschichtungsmasse oder zu untersuchender Flüssigkeit eingesetzt.

Zur Herstellung des erfindungsgemäßen diagnostischen Mittels werden z. B. saugfähige Träger, vorzugsweise Filterpapier, Cellulose oder Kunstfaservliese, mit Lösungen der erforderlichen, üblicherweise zur Herstellung von Teststreifen verwendeten Reagenzien (Substrat, Puffer, Aktivatoren, gegebenenfalls Netzmittel, Komplexbildner, Oxidationsmittel etc.) in leichtflüchtigen Lösungsmitteln, wie z. B. Wasser, Methanol, Ethanol oder Aceton, imprägniert. Dies geschieht zweckmäßig in zwei Schritten:

Zunächst wird mit einer wäßrigen Lösung imprägniert, die den Puffer und gegebenenfalls wasserlösliche Aktivatoren, enthält. Danach wird mit einer Lösung, welche die Esterase- oder Protease-Substrate, gegebenenfalls nicht wasserlösliche Aktivatoren, sowie andere nicht wasserlösliche Zusatzstoffe enthält, imprägniert. In speziellen Fällen kann auch die umgekehrte Imprägnierfolge angewandt werden.

Die fertigen Testpapiere können als solche verwendet werden oder in an sich bekannter Weise an Griffen angeklebt oder vorzugsweise zwischen Kunststoffen und feinmaschigen Netzwerken gemäß DBP 2 118 455 eingesiegelt werden.

Zur Herstellung filmbeschichterer Teststreifen werden sämtliche Reagenzien in die Lösung oder Dispersion einer filmbildenden Substanz, wie z. B. Polyvinylester oder Polyamid, eingetragen und homogen vermischt. Das Gemsich wird in dünner Schicht auf einen Kunststoffträger gestrichen und getrocknet. Die erfindungsgemäßen filmbeschichteten Teststreifen werden nach dem Trocknen geschnitten und können als solche verwendet werden oder in an sich bekannter Weise an Griffen angeklebt werden oder z. B. zwischen Kunststoffen und feinmaschigen Netzwerken gemäß DBP 2 118 455 eingesiegelt werden.

Das erfindungsgemäße diagnostische Mittel zum Nachweis esterolytischer und/oder proteolytischer Enzyme in Form von Pulvermischungen oder Reagenztabletten läßt sich herstellen, indem die oben angeführten Bestandteile des Testes mit üblichen galenischen Zusatzstoffen versetzt und granuliert werden. Zusatzstoffe dieser Art sind z. B. Kohlenhydrate, wie z. B. Mono-, Oligo- oder Polysaccharide, oder Zuckeralkohole, wie z. B. Mannit, Sorbit oder Xylit, oder andere lösliche inerte Verbindungen, wie Polyethylenglykole oder Polyvinylpyrrolidon. Die Pulvermischungen oder Reagenztabletten weisen im allgemeinen ein Endgewicht von ungefähr 50–200 mg, vorzugsweise 50–80 mg, auf.

Zur Herstellung von Lyophilisaten im Gesamtgewicht von jeweils etw 5–20 mg, vorzugsweise etwa 10 mg, wird eine Lösung gefriergetrocknet, die neben sämtlichen für den Test benötigten Reagenzien übliche Gerüstbildner, wie z. B. Polyvinylpyrrolidon, und evtl. weitere Füllstoffe, wie z. B. Mannit, Sorbit oder Xylit, enthält.

Das erfindungsgemäße Diagnostische Mittel in Form einer Lösung enthält vorzugsweise sämtliche für den Test benötigten Reagenzien. Als Lösungsmittel kommen Wasser oder Gemische von Wasser mit einem wasserlöslichen organischen Lösungsmittel, wie z. B. Methanol, Ethanol, Aceton oder Dimethylformamid, in Frage. Aus Haltbarkeitsgründen kann es vorteilhaft sein, die für den Test benötigten Reagenzien auf zwei oder mehr Lösungen zu verteilen, die erst bei der eigentlichen Untersuchung zusammengegeben werden.

Das so hergestellte diagnostische Mittel ermöglicht es, in den zu untersuchenden Körperflüssigkeiten die Leukozyten rasch und einfach über eine Farbbildung bzw. eine Farbänderung nachzuweisen. Im Vergleich zu den diagnostischen Mitteln der Anmeldungen EP-A-0 007 407, EP-A-0 008 428 und EP-A-0 012 957 sind dabei bei Anwendung der erfindungsgemäßen Aktivatoren deutlich verkürzte Reaktionszeiten zu beobachten.

Die vorliegende Erfindung wird durch die nachfolgenden Beispiele näher erläutert.

## Beispiel 1

Filterpapier (z. B. Schleicher & Schüll 23 SL) wird nacheinander mit den folgenden Lösungen imprägniert und dann bei 60° C bzw. Raumtemperatur getrocknet.

Lösung 1

Tris-(hydroxymethyl)-amino-methan-HCl-Puffer, 0,2 mol/Liter, pH 9,0, in Wasser.

Lösung 2

Substratlösungen $10^{-3}$ mol/Liter in Aceton.

Die erfindungsgemäßen Aktivatoren werden — je nach Löslichkeit — der Lösung 1 oder Lösung 2 zugesetzt, so daß bei den Aktivatoren der allgemeinen Formeln I, II und IV Endkonzentrationen von $10^{-2}$ mol/Liter Imprägnierlösung, bei den Aktivatoren der allgemeinen Formel III Endkonzentrationen von 2% (w/v) der Imprägnierlösung resultieren.

In der Tabelle 1 sind die Versuchsergebnisse mit folgenden Esterase- oder Protease-Substraten zusammengestellt:

A: Diacetyl-3',3''-dibrom-5',5''-dichlor-phenolsulfonphthalein,
B: Di-acetyl-4,5,6,7,3',5',3'',5''-octabrom-phenolsulfonphthalein,
C: Di-(N-benzylcarbonyl-L-alanyl)-3',5',3'',5''-tetrabrom-phenolsulfonphthalein,
D: Di-(N-benzyloxycarbonyl-L-phenylalanyl)-3',5',3'',5''-tetrabrom-phenolsulfonphthalein.

In der Tabelle 1 sind die Reaktionszeiten aufgeführt, die vom Eintauchen der Teststreifen in eine Standardlösung von 5000 Leukozyten/µl isotonischer Kochsalzlösung bis zur ersten deutlichen Farbreaktion verstreichen. Als Bezugswerte dienen die Reaktionszeiten der jeweiligen Rezepturen ohne Aktivatorzusätze. Der Farbumschlag bei den vier hier untersuchten Verbindungen erfolgt von farblos nach tiefblau.

Tabelle 1

| Aktivatoren | | Reaktionszeiten für Substrat A | B | C | D |
|---|---|---|---|---|---|
| Vergleichsrezeptur ohne Aktivator | | 210 sec. | 90 sec. | 600 sec. | 550 sec. |
| 1. | Pyridin | 110 sec. | 70 sec. | 410 sec. | 450 sec. |
| 2. | 3-Äthyl-pyridin | 95 sec. | 65 sec. | 440 sec. | 470 sec. |
| 3. | 4-Methoxy-pyridin | 110 sec. | 65 sec. | 470 sec. | 450 sec. |
| 4. | Chinolin | 75 sec. | 50 sec. | 320 sec. | 340 sec. |
| 5. | 2-Methyl-chinolin | 45 sec. | 55 sec. | 300 sec. | 350 sec. |
| 6. | 4-Brom-chinolin | 60 sec. | 60 sec. | 370 sec. | 330 sec. |
| 7. | 3-Methoxy-chinolin | 50 sec. | 50 sec. | 310 sec. | 380 sec. |
| 8. | Isochinolin | 85 sec. | 55 sec. | 350 sec. | 380 sec. |
| 9. | Benzo-[b]-chinolin = Acridin | 90 sec. | 65 sec. | 280 sec. | 330 sec. |
| 10. | Benzo-[c]-chinolin = Phenanthridin | 75 sec. | 60 sec. | 260 sec. | 360 sec. |
| 11. | 2-Methyl-phenanthridin | 100 sec. | 65 sec. | 250 sec. | 370 sec. |
| 12. | 2-Äthyl-phenanthridin | 80 sec. | 70 sec. | 300 sec. | 360 sec. |
| 13. | 2-Propyl-phenanthridin | 80 sec. | 60 sec. | 280 sec. | 330 sec. |
| 14. | 2-Methoxy-phenanthridin | 85 sec. | 60 sec. | 260 sec. | 350 sec. |
| 15. | Benzo-[f]-chinolin | 90 sec. | 40 sec. | 310 sec. | 310 sec. |
| 16. | Benzo-[h]-chinolin | 90 sec. | 55 sec. | 290 sec. | 340 sec. |
| 17. | 1,7-Phenanthrolin | 75 sec. | 65 sec. | 340 sec. | 410 sec. |
| 18. | 4,7-Phenanthrolin | 80 sec. | 70 sec. | 270 sec. | 380 sec. |
| 19. | 4-Aza-fluoren | 120 sec. | 50 sec. | 240 sec. | 290 sec. |
| 20. | Chinin | 110 sec. | 70 sec. | 350 sec. | 330 sec. |
| 21. | Chinidin | 70 sec. | 65 sec. | 375 sec. | 280 sec. |
| 22. | Cinchonin | 60 sec. | 50 sec. | 340 sec. | 300 sec. |
| 23. | Cinchonidin | 65 sec. | 55 sec. | 390 sec. | 320 sec. |
| 24. | Cuprein | 70 sec. | 60 sec. | 400 sec. | 305 sec. |
| 25. | 2-[Phenyl]-vinyl-pyridin-(2′) | 115 sec. | 70 sec. | 370 sec. | 270 sec. |
| 26. | 2-[Pyridyl-(3″)]-vinyl-pyridin-(2′) | 145 sec. | 65 sec. | 430 sec. | 250 sec. |
| 27. | 2-[Pyridyl-(4″)]-vinyl-pyridin-(4′) | 140 sec. | 55 sec. | 350 sec. | 280 sec. |
| 28. | 2-[Furyl-(2″)]-vinyl-pyridin-(2′) | 150 sec. | 70 sec. | 410 sec. | 250 sec. |

Fortsetzung

| Aktivatoren | | Reaktionszeiten für Substrat | | | |
|---|---|---|---|---|---|
| | | A | B | C | D |
| 29. | Imidazol | 90 sec. | 55 sec. | 380 sec. | 210 sec. |
| 30. | Histamin | 110 sec. | 60 sec. | 440 sec. | 290 sec. |
| 31. | (Imidazolyl-4)-essigsäure | 150 sec. | 75 sec. | 450 sec. | 350 sec. |
| 32. | β-(Imidazolyl-4-)-propionsäure | 170 sec. | 70 sec. | 490 sec. | 380 sec. |
| 33. | L-Histidin | 130 sec. | 80 sec. | 465 sec. | 320 sec. |
| 34. | Octanol-(1) | 150 sec. | 75 sec. | 500 sec. | 460 sec. |
| 35. | Decanol-(1) | 130 sec. | 70 sec. | 480 sec. | 420 sec. |
| 36. | Tetradecanol-(1) | 105 sec. | 60 sec. | 420 sec. | 400 sec. |
| 37. | Cyclooctanol | 125 sec. | 80 sec. | 530 sec. | 480 sec. |
| 38. | Cyclododecanol | 90 sec. | 55 sec. | 490 sec. | 470 sec. |
| 39. | Citronellol | 100 sec. | 40 sec. | 430 sec. | 440 sec. |
| 40. | Farnesol | 130 sec. | 50 sec. | 440 sec. | 410 sec. |
| 41. | Phytol | 135 sec. | 55 sec. | 430 sec. | 450 sec. |
| 42. | Tri-kalium-hexacyano-ferrat-II | 160 sec. | 70 sec. | 550 sec. | 480 sec. |
| 43. | Di-kalium-tetracyano-nickelat-II | 160 sec. | 80 sec. | 530 sec. | 450 sec. |
| 44. | Di-natrium-pentacyano-nitrosyl-ferrat-II | 150 sec. | 65 sec. | 470 sec. | 460 sec. |

Ähnliche Versuchsergebnisse werden erzielt, wenn man anstelle der Substrate A, B, C und D andere Sulfonphthalein-Ester der Anmeldung EP-A-0 007 407 und /oder anstelle der Standardlösung von 5000 Leukozyten/µl isotonischer Kochsalzlösung leukozytenhaltige Harne einsetzt.

## Beispiel 2

Filterpapier (z. B. Schleicher & Schüll 23 SL) wird nacheinander mit den folgenden Lösungen imprägniert und dann bei 60° C bzw. Raumtemperatur getrocknet.

Lösung 1
Tris-(hydroxymethyl)-aminomethan-HCl-Puffer, 0,2 mol/Liter, pH 7,0, in Wasser (für Substrate E, F und G), bzw.
Tris-(hydroxymethyl)-amino-methan-HCl-Puffer, 0,2 mol/Liter, pH 8,0, in Wasser (für Substrat H).

Lösung 2
Substratlösungen $10^{-3}$ mol/Liter in Aceton.

Aktivatorzugaben und Versuchsdurchführung erfolgten wie in Beispiel 1.
In Tabelle 2 sind die Versuchsergebnisse der folgenden Protease-Substrate zusammengestellt:

E: Thiazol-2-azo-4′-[1′-(N-benzyloxycarbonyl-L-alanyloxy)-5′-methoxy-naphthalin] (Farbumschlag der Testpapiere von rosa nach rot).
F: 6-Methoxy-benzothiazol-2-azo-2′-[1′-(N-benzyloxycarbonyl-L-alanyloxy)-naphthalin] (Farbumschlag der Testpapiere von rosa nach rot).
G: 2,4-Dinitro-benzol-azo-4′-[1′-(N-benzyloxycarbonyl-L-alanyloxy)-benzol] (Farbumschlag der Testpapiere von gelb nach rot-violett).
H: 2,5-Dimethoxy-benzol-azo-4′-[1′-(N-benzyloxycarbonyl-L-alanyloxy)-naphthalin] (Farbumschlag der Testpapiere von hellorange nach rot).

Es sind die Reaktionszeiten aufgeführt, die vom Eintauchen der Teststreifen in eine Standardlösung von 5000 Leukozyten/µl isotonischer Kochsalzlösung bis zur ersten deutlichen Farbreaktion verstreichen. Als Bezugswerte dienen die Reaktionszeiten der jeweiligen Rezepturen ohne Aktivatorzusätze.

Tabelle 2

| Aktivatoren | Reaktionszeiten für Substrat | | | |
|---|---|---|---|---|
| | E | F | G | H |
| Vergleichsreptur ohne Aktivator | 60 sec. | 180 sec. | 160 sec. | 70 sec. |
| 1. Pyridin | 50 sec. | 150 sec. | 130 sec. | 55 sec. |
| 2. 2-Methyl-pyridin | 45 sec. | 140 sec. | 135 sec. | 50 sec. |
| 3. 2-Brom-pyridin | 50 sec. | 150 sec. | 125 sec. | 45 sec. |
| 4. 3,5-Dichlor-pyridin | 45 sec. | 160 sec. | 130 sec. | 50 sec. |
| 5. Chinolin | 50 sec. | 110 sec. | 95 sec. | 45 sec. |
| 6. 8-Methyl-chinolin | 45 sec. | 130 sec. | 110 sec. | 40 sec. |
| 7. 2-Chlor-chinolin | 40 sec. | 130 sec. | 110 sec. | 35 sec. |
| 8. 6-Äthoxy-chinolin | 35 sec. | 150 sec. | 120 sec. | 40 sec. |
| 9. 2-Methyl-4-methoxy-chinolin | 40 sec. | 120 sec. | 105 sec. | 40 sec. |
| 10. Isochinolin | 40 sec. | 130 sec. | 110 sec. | 40 sec. |
| 11. 1-Methyl-isochinolin | 45 sec. | 100 sec. | 120 sec. | 35 sec. |
| 12. 4-Brom-isochinolin | 45 sec. | 140 sec. | 115 sec. | 40 sec. |
| 13. 7-Methoxy-isochinolin | 50 sec. | 130 sec. | 130 sec. | 45 sec. |
| 14. Benzo-[b]-chinolin = Acridin | 30 sec. | 150 sec. | 60 sec. | 45 sec. |
| 15. Benzo-[c]-chinolin = Phenanthridin | 35 sec. | 120 sec. | 80 sec. | 40 sec. |
| 16. 2-Methyl-phenanthridin | 40 sec. | 140 sec. | 90 sec. | 40 sec. |
| 17. Benzo-[f]-chinolin | 50 sec. | 130 sec. | 70 sec. | 30 sec. |
| 18. 2-Isopropyl-benzo-[f]-chinolin | 45 sec. | 130 sec. | 95 sec. | 35 sec. |
| 19. 3-Methyl-benzo-[f]-chinolin | 40 sec. | 125 sec. | 80 sec. | 40 sec. |
| 20. 2,4-Dimethyl-benzo-[f]-chinolin | 40 sec. | 110 sec. | 90 sec. | 40 sec. |

Fortsetzung

| Aktivatoren | | Reaktionszeiten für Substrat E | F | G | H |
|---|---|---|---|---|---|
| 21. | Benzo-[g]-chinolin | 40 sec. | 90 sec. | 70 sec. | 35 sec. |
| 22. | 4-Methyl-benzo-[g]-chinolin | 40 sec. | 100 sec. | 80 sec. | 40 sec. |
| 23. | Benzo-[h]-chinolin | 35 sec. | 70 sec. | 50 sec. | 30 sec. |
| 24. | 1,7-Phenanthrolin | 45 sec. | 120 sec. | 120 sec. | 40 sec. |
| 25. | 4,7-Phenanthrolin | 40 sec. | 105 sec. | 105 sec. | 45 sec. |
| 26. | 1,10-Phenanthrolin | 45 sec. | 130 sec. | 110 sec. | 45 sec. |
| 27. | Chinin | 40 sec. | 120 sec. | 60 sec. | 35 sec. |
| 28. | Cinchonin | 45 sec. | 100 sec. | 90 sec. | 30 sec. |
| 29. | Cuprein | 40 sec. | 140 sec. | 105 sec. | 30 sec. |
| 30. | 2-[Phenyl]-vinyl-pyridin-(2′) | 35 sec. | 125 sec. | 120 sec. | 50 sec. |
| 31. | Bis-[2-(Phenyl)-vinyl]-pyridin-(2′,4′) | 30 sec. | 150 sec. | 140 sec. | 60 sec. |
| 32. | 2-[Furyl-(2″)]-vinyl-pyridin-(2′) | 30 sec. | 120 sec. | 90 sec. | 55 sec. |
| 33. | Imidazol | 45 sec. | 140 sec. | 120 sec. | 40 sec. |
| 34. | 1-Äthyl-imidazol | 40 sec. | 130 sec. | 130 sec. | 45 sec. |
| 35. | 1-Phenyl-imidazol | 35 sec. | 120 sec. | 120 sec. | 40 sec. |
| 36. | 1-(4′-Hydroxy-phenyl)-imidazol | 45 sec. | 140 sec. | 135 sec. | 50 sec. |
| 37. | Histamin | 50 sec. | 130 sec. | 140 sec. | 55 sec. |
| 38. | β-(Imidazolyl-4)-propionsäure | 50 sec. | 150 sec. | 140 sec. | 50 sec. |
| 39. | β-(Imidazolyl-4)-acrylsäure | 50 sec. | 160 sec. | 135 sec. | 55 sec. |
| 40. | L-Histidin | 45 sec. | 150 sec. | 125 sec. | 50 sec. |
| 41. | N-α-Acetyl-L-histidin | 45 sec. | 140 sec. | 130 sec. | 40 sec. |
| 42. | Heptanol-(1) | 30 sec. | 130 sec. | 100 sec. | 40 sec. |
| 43. | Octanol-(1) | 30 sec. | 120 sec. | 95 sec. | 35 sec. |
| 44. | Decanol-(1) | 20 sec. | 90 sec. | 80 sec. | 30 sec. |
| 45. | Dodecanol-(1) | 20 sec. | 80 sec. | 70 sec. | 30 sec. |
| 46. | Hexadecanol-(1) | 25 sec. | 105 sec. | 80 sec. | 40 sec. |
| 47. | Eicosanol-(1) | 30 sec. | 120 sec. | 110 sec. | 45 sec. |
| 48. | Cyclohexanol | 35 sec. | 110 sec. | 105 sec. | 40 sec. |

Fortsetzung

| Aktivatoren | | Reaktionszeiten für Substrat | | | |
|---|---|---|---|---|---|
| | | E | F | G | H |
| 49. | Cyclodecanol | 25 sec. | 95 sec. | 85 sec. | 30 sec. |
| 50. | Cyclododecanol | 25 sec. | 120 sec. | 90 sec. | 25 sec. |
| 51. | Cycloheptadecanol | 30 sec. | 130 sec. | 120 sec. | 30 sec. |
| 52. | Cycloheptadecen-(9)-ol-(1) | 30 sec. | 110 sec. | 100 sec. | 35 sec. |
| 53. | Citronellol | 25 sec. | 100 sec. | 70 sec. | 25 sec. |
| 54. | Linalool | 20 sec. | 105 sec. | 60 sec. | 30 sec. |
| 55. | Farnesol | 30 sec. | 110 sec. | 75 sec. | 25 sec. |
| 56. | cis-Octadecen-(9)-ol-(1) | 25 sec. | 90 sec. | 85 sec. | 40 sec. |
| 57. | Phytol | 30 sec. | 80 sec. | 65 sec. | 30 sec. |
| 58. | Pentadiol-(1,5) | 35 sec. | 130 sec. | 120 sec. | 45 sec. |
| 59. | Heptandiol-(1,7) | 40 sec. | 150 sec. | 125 sec. | 40 sec. |
| 60. | Decandiol-(1,10) | 30 sec. | 135 sec. | 140 sec. | 50 sec. |
| 61. | Dodecandiol-(1,12) | 40 sec. | 160 sec. | 130 sec. | 50 sec. |
| 62. | Tri-kalium-hexacyano-ferrat-III | 45 sec. | 160 sec. | 135 sec. | 55 sec. |
| 63. | Tetra-kalium-hexacyano-ferrat-II | 40 sec. | 160 sec. | 140 sec. | 50 sec. |
| 64. | Di-natrium-pentacyano-nitrosyl-ferrat-II | 40 sec. | 140 sec. | 130 sec. | 40 sec. |
| 65. | Tri-kalium-pentacyano-nitrosyl-chromat-I | 50 sec. | 145 sec. | 130 sec. | 50 sec. |
| 66. | Tri-kalium-pentacyano-nitrosyl-kobaltat-I | 50 sec. | 160 sec. | 145 sec. | 45 sec. |

Ähnliche Versuchsergebnisse werden erreicht, wenn man anstelle der Substrate E, F, G und H andere Azo-Farbstoff-Ester der Anmeldung EP-A-0 008 428 und/oder anstelle der Standardlösung von 5000 Leukozyten/µl isotonischer Kochsalzlösung leukozytenhaltige Harne einsetzt.

## Beispiel 3

Filterpapier (z. B. Schleicher & Schüll 23 SL) wird nacheinander mit den folgenden Lösungen imprägniert und dann bei 60° C bzw. Raumtemperatur getrocknet.

Lösung 1
Natrium-tetraborat-HCl-Puffer, 0,2 mol/Liter, pH 8,0, in Wasser.

Lösung 2
Substratlösung $10^{-3}$ mol/Liter in Aceton.

Aktivatorzugaben und Versuchsdurchführung erfolgten wie in Beispiel 1.
In Tabelle 3 sind die Versuchsergebnisse der folgenden Protease-Substrate zusammengestellt:

I: 3-[N-(Diphenylcarbamoyl)-L-alanyloxy]-indol
J: 3-[N-(5′,5′-Dimethyl-3′-oxo-cyclohexen-1′-yl)-L-alanyloxy]-indol
K: 3-[N-(Benzyloxycarbonyl)-L-alanyloxy]-indol.

Es sind die Reaktionszeiten aufgeführt, die vom Eintauchen der Teststreifen in eine Standardlösung von 5000 Leukozyten/µl isotonischer Kochsalzlösung bis zur ersten deutlichen Farbreaktion verstreichen. Als Bezugswerte dienen die Reaktionszeiten der jeweiligen Rezepturen ohne Aktivatorzusätze.
Die mit den drei Substraten gefertigten Testpapiere ergeben nach Eintauchen in leukozytenhaltige Lösungen Farbumschläge von farblos nach tiefblau.

Tabelle 3

| Aktivatoren | Reaktionszeiten für Substrat I | J | K |
|---|---|---|---|
| Vergleichsrezeptur ohne Aktivator | 300 sec. | 180 sec. | 60 sec. |
| 1. Pyridin | 220 sec. | 140 sec. | 45 sec. |
| 2. 2-Brom-pyridin | 205 sec. | 130 sec. | 40 sec. |
| 3. 2,6-Dimethyl-4-äthoxy-pyridin | 160 sec. | 110 sec. | 40 sec. |
| 4. Chinolin | 185 sec. | 90 sec. | 35 sec. |
| 5. 2-Methyl-chinolin | 190 sec. | 105 sec. | 40 sec. |
| 6. 7-Isopropyl-chinolin | 165 sec. | 80 sec. | 35 sec. |
| 7. 3-Methoxy-chinolin | 150 sec. | 75 sec. | 30 sec. |
| 8. 2-Methyl-6-brom-chinolin | 170 sec. | 80 sec. | 40 sec. |
| 9. 5,7-Dibrom-8-methoxy-chinolin | 195 sec. | 90 sec. | 40 sec. |
| 10. Isochinolin | 150 sec. | 80 sec. | 40 sec. |
| 11. 3-Propyl-isochinolin | 170 sec. | 75 sec. | 35 sec. |
| 12. 7-Methyl-isochinolin | 145 sec. | 105 sec. | 30 sec. |
| 13. 1-Chlor-isochinolin | 180 sec. | 90 sec. | 35 sec. |
| 14. 1-Methoxy-3-chlor-isochinolin | 155 sec. | 95 sec. | 40 sec. |
| 15. 1-Chlor-4-methyl-5-methoxy-isochinolin | 190 sec. | 80 sec. | 35 sec. |
| 16. Benzo-[b]-chinolin = Acriain | 170 sec. | 90 sec. | 40 sec. |
| 17. Benzo-[c]-chinolin- = Phenanthridin | 130 sec. | 110 sec. | 35 sec. |
| 18. 2-Äthyl-phenanthridin | 160 sec. | 100 sec. | 30 sec. |
| 19. 2-Methoxy-phenanthridin | 145 sec. | 120 sec. | 35 sec. |
| 20. Benzo-[f]-chinolin | 130 sec. | 105 sec. | 35 sec. |

Fortsetzung

| Aktivatoren | | Reaktionszeiten für Substrat | | |
|---|---|---|---|---|
| | | I | J | K |
| 21. | Benzo-[g]-chinolin | 140 sec. | 130 sec. | 45 sec. |
| 22. | 2,4-Dimethyl-benzo-[g]-chinolin | 135 sec. | 110 sec. | 40 sec. |
| 23. | Benzo-[h]-chinolin | 110 sec. | 70 sec. | 40 sec. |
| 24. | 1,7-Phenanthrolin | 165 sec. | 130 sec. | 45 sec. |
| 25. | 2-Methyl-1,7-Phenanthrolin | 130 sec. | 110 sec. | 45 sec. |
| 26. | 2,8-Dimethyl-1,7-Phenanthrolin | 145 sec. | 105 sec. | 40 sec. |
| 27. | 4,7-Phenanthrolin | 120 sec. | 120 sec. | 35 sec. |
| 28. | 3-Methyl-4,7-Phenanthrolin | 145 sec. | 95 sec. | 40 sec. |
| 29. | 3,8-Dimethyl-4,7-Phenanthrolin | 160 sec. | 80 sec. | 35 sec. |
| 30. | 1,10-Phenanthrolin | 170 sec. | 90 sec. | 35 sec. |
| 31. | 2,9-Dimethyl-1,10-Phenanthrolin | 145 sec. | 120 sec. | 45 sec. |
| 32. | 4-Aza-fluoren | 90 sec. | 60 sec. | 35 sec. |
| 33. | Chinin | 150 sec. | 90 sec. | 30 sec. |
| 34. | Cinchonidin | 125 sec. | 85 sec. | 30 sec. |
| 35. | Cuprein | 160 sec. | 95 sec. | 35 sec. |
| 36. | 2-[4″-Methoxy-phenyl]-vinyl-pyridin-(2′) | 95 sec. | 90 sec. | 45 sec. |
| 37. | 2-[4″-(N,N-Dimethylamino)-phenyl]-vinyl-pyridin-(2′) | 110 sec. | 110 sec. | 45 sec. |
| 38. | Bis-[2-(Phenyl)-vinyl]-pyridin-(2′,4′) | 120 sec. | 95 sec. | 40 sec. |
| 39. | 2-[Naphthyl-(1″)]-vinyl-pyridin-(2′) | 105 sec. | 90 sec. | 40 sec. |
| 40. | 2-[Pyridyl-(2″)]-vinyl-pyridin-(2′) | 80 sec. | 80 sec. | 30 sec. |
| 41. | 2-[Pyridyl-(4″)]-vinyl-pyridin-(2′) | 60 sec. | 50 sec. | 25 sec. |
| 42. | 2-[Pyridyl-(3″)]-vinyl-pyridin-(3′) | 75 sec. | 70 sec. | 30 sec. |
| 43. | 2-[Pyridyl-(3″)]-vinyl-pyridin-(4′) | 95 sec. | 90 sec. | 25 sec. |
| 44. | 2-[Thienyl-(2″)]-vinyl-pyridin-(4′) | 80 sec. | 75 sec. | 35 sec. |

Fortsetzung

| Aktivatoren | | Reaktionszeiten für Substrat I | J | K |
|---|---|---|---|---|
| 45. | Imidazol | 175 sec. | 120 sec. | 35 sec. |
| 46. | 1-Phenyl-imidazol | 180 sec. | 140 sec. | 40 sec. |
| 47. | Histamin | 230 sec. | 150 sec. | 45 sec. |
| 48. | N-α-Acetyl-histamin | 205 sec. | 130 sec. | 40 sec. |
| 49. | (Imidazolyl-4)-essigsäure | 220 sec. | 150 sec. | 50 sec. |
| 50. | L-Histidin | 195 sec. | 120 sec. | 45 sec. |
| 51. | N-α-Acetyl-L-histidin | 165 sec. | 110 sec. | 40 sec. |
| 52. | Hexanol-(1) | 95 sec. | 95 sec. | 30 sec. |
| 53. | Octanol-(1) | 90 sec. | 100 sec. | 30 sec. |
| 54. | Nonanol-(1) | 90 sec. | 80 sec. | 30 sec. |
| 55. | Decanol-(1) | 80 sec. | 60 sec. | 25 sec. |
| 56. | Dodecanol-(1) | 75 sec. | 90 sec. | 25 sec. |
| 57. | Pentadecanol-(1) | 100 sec. | 80 sec. | 30 sec. |
| 58. | Heptadecanol-(1) | 80 sec. | 105 sec. | 35 sec. |
| 59. | Octadecanol-(1) | 90 sec. | 95 sec. | 40 sec. |
| 60. | Nonadecanol-(1) | 85 sec. | 120 sec. | 40 sec. |
| 61. | Docosanol-(1) | 85 sec. | 110 sec. | 40 sec. |
| 62. | Cyclohexen-(1)-ol-(1) | 95 sec. | 130 sec. | 35 sec. |
| 63. | Cyclononanol | 80 sec. | 120 sec. | 35 sec. |
| 64. | Cyclodecanol | 80 sec. | 115 sec. | 40 sec. |
| 65. | Cycloheptadecanol | 90 sec. | 130 sec. | 40 sec. |
| 66. | Geraniol | 75 sec. | 85 sec. | 30 sec. |
| 67. | Nerol | 90 sec. | 70 sec. | 35 sec. |
| 68. | Linalool | 80 sec. | 80 sec. | 30 sec. |
| 69. | Nerolidol | 105 sec. | 90 sec. | 25 sec. |
| 70. | cis-Octadecen-(9)-ol-(1) | 110 sec. | 115 sec. | 25 sec. |
| 71. | Hexandiol-(1,6) | 130 sec. | 130 sec. | 45 sec. |
| 72. | Octandiol-(1,8) | 115 sec. | 125 sec. | 40 sec. |

Fortsetzung

| Aktivatoren | | Reaktionszeiten für Substrat | | |
|---|---|---|---|---|
| | | I | J | K |
| 73. | Nonandiol-(1,9) | 140 sec. | 130 sec. | 45 sec. |
| 74. | Decandiol-(1,10) | 130 sec. | 115 sec. | 50 sec. |
| 75. | Tetra-kalium-hexacyano-ferrat-II | 230 sec. | 160 sec. | 50 sec. |
| 76. | Tri-natrium-octacyano-molybdat-V | 205 sec. | 150 sec. | 50 sec. |
| 77. | Tri-kalium-pentacyano-nitrosyl-ferrat-II | 180 sec. | 150 sec. | 45 sec. |
| 78. | Tri-kalium-pentacyano-nitrosyl-manganat-I | 210 sec. | 145 sec. | 40 sec. |
| 79. | Penta-kalium-pentacyano-nitrosyl-vanadat-I | 220 sec. | 155 sec. | 45 sec. |

Ähnliche Versuchsergebnisse werden mit anderen Indoxyl-Estern der Anmeldung EP-A-0 012 957 und/oder leukozytenhaltigen Harnen anstelle der Standardlösung von 5000 Leukozyten/µl isotonischer Kochsalzlösung erreicht.

## Beispiel 4

Filterpapier (z. B. Schleicher & Schüll 23 SL) wird nacheinander mit den folgenden Lösungen imprägniert und dann bei 60° C bzw. Raumtemperatur getrocknet.

Lösung 1
Tris-(hydroxymethyl)-amino-methan-HCl-Puffer, 0,2 mol/Liter, pH 9,0, in Wasser.

Lösung 2
Di-acetyl-3',5',3'',5''-tetrabrom-phenylsulfonphthalein, $10^{-3}$ mol/Liter in Aceton.

Die erfindungsgemäßen Aktivatoren werden einzeln oder als Gemisch je nach Löslichkeit der Lösung 1 und/oder Lösung 2 zugesetzt, so daß bei den einzelnen Aktivatoren der allgemeinen Formeln I, II und IV jeweils Endkonzentrationen von $10^{-2}$ mol/Liter Imprägnierlösung, bei den Aktivatoren der allgemeinen Formel III Endkonzentrationen von 2% (w/v) der Imprägnierlösung resultieren.
In Tabelle 4 sind die Reaktionszeiten aufgeführt, die vom Eintauchen der Teststreifen in eine Standardlösung von 5000 Leukozyten/µl isotonischer Kochsalzlösung bis zur ersten deutlichen Farbreaktion verstreichen. Als Bezugswert dient die Reaktionszeit der Rezeptur ohne Aktivatorzusätze.
Die Testpapiere verfärben sich beim Eintauchen in leukozytenhaltige Lösungen von farblos nach tiefblau.

Tabelle 4

| Aktivatoren | | Reaktionszeiten |
|---|---|---|
| Vergleichsrezeptur ohne Aktivatoren | | 160 sec. |
| 1. | 2-Methyl-chinolin | 110 sec. |
| 2. | Tetradecanol-(1) | 90 sec. |
| Aktivatoren 1. und 2. | | 60 sec. |
| 3. | 1,7-Phenanthrolin | 80 sec. |
| 4. | Tetra-kalium-hexacyano-ferrat-II | 140 sec. |
| Aktivatoren 3. und 4. | | 70 sec. |
| 5. | Cinchonin | 90 sec. |
| 6. | 2-[Phenyl]-vinyl-pyridin-(2') | 105 sec. |
| Aktivatoren 5. und 6. | | 65 sec. |

Ähnliche Versuchsergebnisse werden leukozytenhaltigen Harnen anstelle der Standardlösung von 5000 Leukozyten/µl isotonischer Kochsalzlösung erreicht.

## Beispiel 5

In der in Beispiel 4 beschriebenen Weise werden Testpapiere mit folgenden Lösungen hergestellt:

Lösung 1
Tris-(hydroxymethyl)-amino-methan-HCl-Puffer, 0,2 ml/Liter, pH 8,0, in Wasser.

Lösung 2
2-Methoxy-4-nitro-benzol-azo-4'-[1'-(N-benzyloxycarbonyl-L-alanyloxy)-naphthalin] $10^{-3}$ mol/Liter in Aceton.

Die Testpapiere verfärben sich beim Eintauchen in leukozytenhaltigen Lösungen von hellorange nach rot.

In Tabelle 5 sind die Versuchsergebnisse festgehalten. Es sind die Reaktionszeiten aufgeführt, die vom Eintauchen der Teststreifen in eine Standardlösung von 5000 Leukozyten/µl isotonischer Kochsalzlösung bis zur ersten deutlichen Farbreaktion verstreichen. Als Bezugswert dient die Reaktionszeit der Rezeptur ohne Aktivatorzusätze.

Tabelle 5

| Aktivatoren | | Reaktionszeiten |
|---|---|---|
| Vergleichsrezeptur ohne Aktivatoren | | 80 sec. |
| 1. | Benzo-[b]-chinolin = Acridin | 60 sec. |
| 2. | cis-Octadecen-(9)-ol-(1) | 40 sec. |
| Aktivatoren 1. und 2. | | 30 sec. |
| 3. | Benzo-[h]-chinolin | 50 sec. |
| 4. | Chinin | 55 sec. |
| Aktivatoren 3. und 4. | | 35 sec. |
| 5. | 1,7-Phenanthrolin | 45 sec. |
| 6. | Farnesol | 30 sec. |
| Aktivatoren 5. und 6. | | 25 sec. |

Ähnliche Versuchsergebnisse werden mit leukozytenhaltigen Harnen anstelle der Standardlösung von 5000 Leukozyten/µl isotonischer Kochsalzlösung erreicht.

## Beispiel 6

In der in Beispiel 4 beschriebenen Weise werden Testpapiere mit folgenden Lösungen hergestellt:

Lösung 1
Natrium-tetraborat-HCl-Puffer, 0,2 mol/Liter, pH 8,0, in Wasser.

Lösung 2
3-[N-(2′-Nitrobenzol-sulfenyl)-L-alanyloxy]-indol
$10^{-3}$ mol/Liter in Aceton.

Die Testpapiere verfärben sich beim Eintauchen in leukozytenhaltige Lösungen von gelb nach grün.

Die Versuchsergebnisse sind in Tabelle 6 festgehalten. Es sind die Reaktionszeiten aufgeführt, die vom Eintauchen der Teststreifen in eine Standardlösung von 5000 Leukozyten/µl isotonischer Kochsalzlösung bis zur ersten deutlichen Farbreaktion verstreichen. Als Bezugswert dient die Reaktionszeit der Rezeptur ohne Aktivatorzusätze.

Tabelle 6

| Aktivatoren | | Reaktionszeiten |
|---|---|---|
| Vergleichsrezeptur ohne Aktivatoren | | 100 sec. |
| 1. | Chinolin | 80 sec. |
| 2. | Cyclododecanol | 70 sec. |
| Aktivatoren 1. und 2. | | 55 sec. |
| 3. | 2-[Furyl-(2″)]-vinyl-pyridin-(2′) | 75 sec. |
| 4. | Phytol | 50 sec. |
| Aktivatoren 3. und 4. | | 40 sec. |
| 5. | 4,7-Phenanthrolin | 85 sec. |
| 6. | Tetra-kalium-hexacyano-ferrat-II | 90 sec. |
| Aktivatoren 5. und 6. | | 70 sec. |

Ähnliche Versuchsergebnisse werden mit leukozytenhaltigen Harnen anstelle der Standardlösung von 5000 Leukozyten/µl isotonischer Kochsalzlösung erreicht.

## Beispiel 7

In der in Beispiel 4 beschriebenen Weise werden Testpapiere mit folgenden Lösungen hergestellt:

Lösung 1
Natrium-tetraborat-HCl-Puffer, 0,2 mol/Liter, pH 8,0, in Wasser.

Lösung 2
3-[N-(Benzoyl)-D,L-alanyloxy]-indol
$10^{-3}$ mol/Liter in Aceton.

Die Testpapiere verfärben sich beim Eintauchen in leukozytenhaltige Lösungen von farblos nach blau.

Die Versuchsergebnisse sind in Tabelle 7 festgehalten. Es sind die Reaktionszeiten aufgeführt, die vom Eintauchen der Teststreifen in eine Standardlösung von 5000 Leukozyten/µl isotonischer Kochsalzlösung bis zur ersten deutlichen Farbreaktion verstreichen. Als Bezugswert dient die Reaktionszeit der Rezeptur ohne Aktivatorzusätze.

Tabelle 7

| Aktivatoren | | Reaktionszeiten |
|---|---|---|
| Vergleichsrezeptur ohne Aktivatoren | | 90 sec. |
| 1. | 2-[Phenyl]-vinyl-pyridin-(2′) | 70 sec. |
| 2. | Hexadecanol-(1) | 55 sec. |
| Aktivatoren 1. und 2. | | 40 sec. |
| 3. | 2-[Pyridyl-(4″)]-vinyl-pyridin-(4′) | 40 sec. |
| 4. | Linalool | 50 sec. |
| Aktivatoren 3. und 4. | | 30 sec. |
| 5. | Cinchonin | 75 sec. |
| 6. | Di-natrium-pentacyano-nitrosyl-ferrat-II | 60 sec. |
| Aktivatoren 5. und 6. | | 40 sec. |

Ähnliche Versuchsergebnisse werden mit leukozytenhaltigen Harnen anstelle der Standardlösung von 5000 Leukozyten/µl isotonischer Kochsalzlösung erreicht.

Beispiel 7a

In der in Beispiel 4 beschriebenen Weise werden Testpapiere mit folgenden Lösungen hergestellt:

Lösung 1
Natrium-tetraborat-HCl-Puffer, 0,2 mol/Liter, pH 8,0, in Wasser.

Lösung 2
3-[N-(Toluol-4′-sulfonyl)-L-alanyloxy]-indol
$10^{-3}$ mol/Liter in Aceton.

Die Testpapiere verfärben sich beim Eintauchen in leukozytenhaltige Lösungen von farblos nach blau.

Die Versuchsergebnisse sind in Tabelle 7a festgehalten. Es sind die Reaktionszeiten aufgeführt, die vom Eintauchen der Teststreifen in eine Standardlösung von 5000 Leukozyten/µl isotonischer Kochsalzlösung bis zur ersten deutlichen Farbreaktion verstreichen. Als Bezugswert dient die Reaktionszeit der Rezeptur ohne Aktivatorzusätze.

Tabelle 7 a

| Aktivatoren | | Reaktionszeiten |
|---|---|---|
| Vergleichsrezeptur ohne Aktivatoren | | 24 sec. |
| 1. | Chinin | 22 sec. |
| 2. | Di-natrium-pentacyano-nitrosyl-ferrat-II | 15 sec. |
| 3. | Decanol-(1) | 18 sec. |
| Aktivatoren 1., 2. und 3. | | 6 sec. |

Ähnliche Versuchsergebnisse werden mit leukozytenhaltigen Harnen anstelle der Standardlösung von 5000 Leukozyten/µl isotonischer Kochsalzlösung erreicht.

Beispiel 8

Filterpapier (z. B. Schleicher & Schüll 23 SL) wird nacheinander mit den folgenden Lösungen imprägniert und dann bei 60° C bzw. Raumtemperatur getrocknet.

Lösung 1
Laurylpyridiniumchlorid, 0,2% in
Tris-(hydroxymethyl)-amino-methan-HCl-Puffer, 0,2 mol/Liter, pH 7,0, in Wasser.

Lösung 2
Thiazol-2-azo-4'-[1'-(N-benzyloxycarbonyl-L-alanyloxy)-naphthalin], $10^{-3}$ mol/Liter in Aceton.

Aktivatorenzugaben und Versuchsdurchführung erfolgten wie in Beispiel 1.

Die Testpapiere verfärben sich beim Eintauchen in leukozytenhaltige Lösungen von rasa nach violett.

In Tabelle 8 sind die Versuchsergebnisse festgehalten. Es sind die Reaktionszeiten aufgeführt, die vom Eintauchen der Teststreifen in eine Standardlösung von 5000 Leukozyten/µl isotonischer Kochsalzlösung bis zur ersten deutlichen Farbreaktion verstreichen. Als Bezugswerte dienen die Reaktionszeiten der jeweiligen Rezepturen ohne Aktivatorzusätze.

Tabelle 8

| Aktivatoren | | Reaktionszeiten |
|---|---|---|
| Vergleichsrezeptur ohne Aktivator | | 70 sec. |
| 1. | Pyridin | 45 sec. |
| 2. | Chinolin | 40 sec. |
| 3. | Benzo-[b]-chinolin = Acridin | 35 sec. |
| 4. | 1,10-Phenanthrolin | 40 sec. |
| 5. | 4-Aza-fluoren | 30 sec. |
| 6. | Chinin | 35 sec. |
| 7. | 2-[Pyridyl-(4")]-vinyl-pyridin-(4') | 30 sec. |
| 8. | Imidazol | 50 sec. |
| 9. | Dodecanol | 30 sec. |
| 10. | Phytol | 25 sec. |
| 11. | Di-natrium-pentacyano-nitrosyl-ferrat-II | 60 sec. |

Ähnliche Versuchsergebnisse werden mit anderen in den Beispielen 1 bis 7 erwähnten Substraten und Aktivatoren und/oder anderen üblichen Netzmitteln erzielt.

Beispiel 9

Filterpapier (z. B. Schleicher & Schüll 23 SL) wird nacheinander mit den folgenden Lösungen imprägniert und dann bei 60° C bzw. Raumtemperatur getrocknet.

Lösung 1
Tris-(hydroxymethyl)-amino-methan-HCl-Puffer, 0,2 mol/Liter, pH 7,0, in Wasser.

Lösung 2
Thiazol-2-azo-1'-[2'-(N-benzyloxycarbonyl-L-alanyloxy)-naphthalin], $10^{-3}$ mol/Liter und Zinkacetat-dihydrat, $10^{-3}$ mol/Liter in Aceton.

Aktivatorenzugaben und Versuchsdurchführung erfolgten wie in Beispiel 1.
Die Testpapiere verfärben sich beim Eintauchen in leukozytenhaltige Lösungen von rosa nach blau-violett.
In Tabelle 9 sind die Versuchsergebnisse festgehalten. Es sind die Reaktionszeiten aufgeführt, die vom Eintauchen der Teststreifen in eine Standardlösung von 5000 Leukozyten/µl isotonischer Kochsalzlösung bis zur ersten deutlichen Farbreaktion verstreichen. Als Bezugswerte dienen die Reaktionszeiten der jeweiligen Rezepturen ohne Aktivatorzusätze.

Tabelle 9

| Aktivatoren | | Reaktionszeiten |
|---|---|---|
| Vergleichsrezeptur ohne Aktivatoren | | 65 sec. |
| 1. | Pyridin | 50 sec. |
| 2. | Chinolin | 45 sec. |
| 3. | Benzo-[b]-chinolin = Acridin | 40 sec. |
| 4. | 1,10-Phenanthrolin | 30 sec. |
| 5. | 4-Aza-fluoren | 35 sec. |
| 6. | Chinin | 30 sec. |
| 7. | 2-[Pyridyl-(4")]-vinyl-pyridin-(4') | 35 sec. |
| 8. | Imidazol | 45 sec. |
| 9. | Dodecanol | 25 sec. |
| 10. | Phytol | 30 sec. |
| 11. | Di-natrium-pentacyano-nitrosyl-ferrat-II | 50 sec. |

Ähnliche Versuchsergebnisse werden mit anderen Azo-Farbstoffestern der Anmeldung EP-A-0 008 428, mit anderen in den Beispielen 1 bis 7 genannten Aktivatoren und/oder anderen üblichen Komplexbildnern erhalten.

## Beispiel 10

Filterpapier (z. B. Schleicher & Schüll 23 SL) wird nacheinander mit den folgenden Lösungen imprägniert und dann bei 60°C bzw. Raumtemperatur getrocknet.

Lösung 1
Kaliumbromat $10^{-2}$ mol/Liter in
Natrium-tetraborat-HCl-Puffer, 0,2 mol/Liter, pH 8,0, in Wasser.

Lösung 2
3-[N-Formyl-L-alanyloxy]-indol, $10^{-3}$ mol/Liter in Aceton.

Aktivatorenzugaben und Versuchsdurchführung erfolgten wie in Beispiel 1.

Die Testpapiere verfärben sich beim Eintauchen in leukozytenhaltige Lösungen von farblos nach blau.

In Tabelle 10 sind die Versuchsergebnisse festgehalten. Es sind die Reaktionszeiten aufgeführt, die vom Eintauchen der Teststreifen in eine Standardlösung von 5000 Leukozyten/µl isotonischer Kochsalzlösung bis zu ersten deutlichen Farbreaktion verstreichen. Als Bezugswerte dienen die Reaktionszeiten der jeweiligen Rezepturen ohne Aktivatorzusätze.

Tabelle 10

| Aktivatoren | | Reaktionszeiten |
|---|---|---|
| Vergleichsrezeptur ohne Aktivatoren | | 120 sec. |
| 1. | Pyridin | 85 sec. |
| 2. | Chinolin | 70 sec. |
| 3. | Benzo-[b]-chinolin = Acridin | 100 sec. |
| 4. | 1,10-Phenanthrolin | 65 sec. |
| 5. | 4-Aza-fluoren | 50 sec. |
| 6. | Chinin | 75 sec. |
| 7. | 2-[Pyridyl-(4″)]-vinyl-pyridin-(4′) | 70 sec. |
| 8. | Imidazol | 85 sec. |
| 9. | Dodecanol | 35 sec. |
| 10. | Phytol | 40 sec. |
| 11. | Di-natrium-pentacyano-nitrosyl-ferrat-II | 90 sec. |

Ähnliche Versuchsergebnisse werden auch mit anderen Indoxyl-Estern der Anmeldung EP-A-0 012 957, mit anderen in den Beispielen 1 bis 7 beschriebenen Aktivatoren und anderen üblichen Oxidationsmitteln erzielt.

**Patentansprüche**

1. Diagnostisches Mittel zum Nachweis esterolytischer und/oder proteolytischer Enzyme insbesondere Leukozyten-Esterasen und/oder -Proteasen in Körperflüssigkeiten, enthaltend ein übliches Esterase- und/oder Protease-Substrat sowie übliche zusätzliche Hilfsstoffe, dadurch gekennzeichnet, daß es zusätzlich ein oder mehrere Aktivatoren enthält, ausgewählt aus der Gruppe der

a) Pyridin-Derivate der allgemeinen Formel I

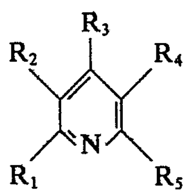

(I)

in der

$R_1$, $R_2$, $R_3$, $R_4$ und $R_5$, die gleich oder verschieden sein können, jeweils Wasserstoff oder Halogen, eine niedere Alkyl- oder eine niedere Alkoxygruppe oder eine Vinylgruppe, die durch einen gegebenenfalls eine oder mehrere niedere Alkoxy-, Amino-, Alkylamino-, Dialkylaminogruppen tragenden Arylrest oder durch einen heterocyclischen Rest substituiert ist, bedeuten,

jeweils zwei benachbarte Substituenten einen gegebenenfalls ein- oder mehrfach durch Halogen, Hydroxygruppe, niedere Alkyl- oder niedere Alkoxygruppen substituierten indeno- oder benzoannellierten Rest, der wiederum einen gegebenenfalls durch eine niedere Alkylgruppe substituierten benzo- oder pyridoannellierten Rest tragen kann, vorstellen können und $R_3$ zusätzlich für eine Vinyl-chinuclidyl-Carbinol-Gruppe stehen kann.

b) Imidazol-Derivate der allgemeinen Formel II

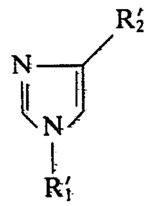

(II)

in der

$R_1'$ Wasserstoff, eine niedere Alkylgruppe oder einen gegebenenfalls durch eine Hydroxy- oder eine Acylgruppe substituierten Arylrest und

$R_2'$ Wasserstoff, eine Aminoalkyl-, N-Acylaminoalkyl- oder einen niederen aliphatischen, gegebenenfalls ungesättigten Carbonsäure-Rest oder einen gegebenenfalls am Stickstoff acylierten niederen aliphatischen α-Aminosäure-Rest,

bedeuten.

c) Alkohole der allgemeinen Formel III

$$X—A—OH \quad (III)$$

in der

X Wasserstoff oder eine Hydroxygruppe und

A einen Kohlenwasserstoffrest

bedeuten.

d) Metallkomplexe der allgemeinen Formel IV

$$D_m[B(CN)_n(NO)_p] \quad (IV)$$

in der

D ein Alkalimetallion,

B ein Schwermetallion,

m eine ganze Zahl von 2 bis 5,

n eine ganze Zahl von 4 bis 8 und

p 0 oder 1

bedeuten, wobei sich die Zahl m aus der Wertigkeit des Schwermetallions und der Zahl n ergibt.

2. Diagnostisches Mittel gemäß Anspruch 1, dadurch gekennzeichnet, daß als zusätzliche Hilfsstoffe Puffer, Komplexbildner, Netzmittel, Oxidationsmittel, Filmbildner, galenische Zusatzstoffe und/oder Gerüstbildner verwendet werden.

3. Diagnostisches Mittel gemäß einem der Ansprüche 1 und 2, dadurch gekennzeichnet, daß als übliche Esterase- und/oder Protease-Substrate Sulfonphthalein-Ester der allgemeinen Formel A

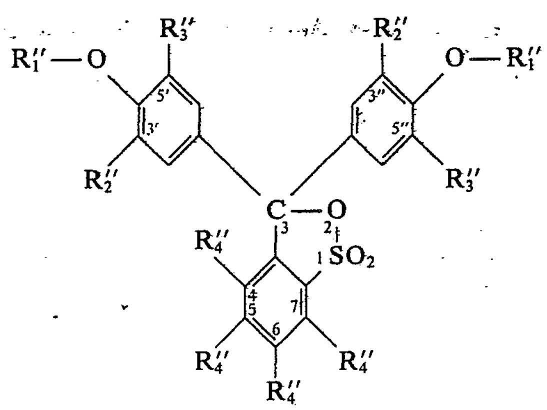

(A)

in der

$R_1''$ einen gegebenenfalls durch Halogen oder eine niedere Alkoxygruppe substituierten

Carbonsäurerest, einen mit einer in der Peptidchemie üblichen Stickstoffschutzgruppe versehenen Aminosäure- oder Peptidrest,

$R_2''$ ein Halogenatom oder eine niedere Alkylgruppe,

$R_3''$ und $R_4''$, die gleich oder verschieden sein können, jeweils ein Wasserstoff- oder ein Halogenatom bedeuten,

Azo-Farbstoff-Ester der allgemeinen Formel B

$$A'—N{=}N—B'(OR)_n \qquad (B)$$

in der

A' einen fünf- oder sechsgliedrigen, gegebenenfalls benzo-annellierten Rest mit ein bis zwei Heteroatomen aus der Gruppe N, S, O, der gegebenenfalls ein- oder mehrfach durch Halogen, niedere Alkyl- und/oder niedere Alkoxygruppen substituiert sein kann oder einen ein- bis dreifach durch niedere Alkyl-, niedere Alkoxy-, Nitro-, Sulfonato- und/oder Acylaminogruppen substituierten Phenylrest,

B' einen gegebenenfalls ein- bis zweifach durch Sulfonato-, niedere Alkoxy- und/oder niedere Alkoxy-poly-alkenoxy-Gruppen substituierten Benzol-, Naphthalin- oder Chinolin-Rest,

R einen Carbonsäurerest oder einen mit einer in der Peptidchemie üblichen Stickstoffschutzgruppe versehenen Aminosäure- oder Peptid-Rest und

n die Zahl 1 oder 2 bedeuten,

oder

Indoxyl-Ester der allgemeinen Formel C

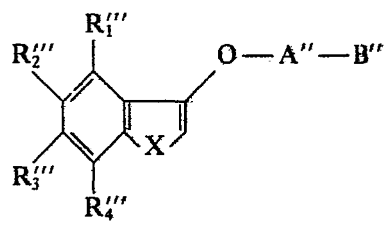

(C)

in der

$R_1'''$, $R_2'''$, $R_3'''$, $R_4'''$, die gleich oder verschieden sein können, jeweils Wasserstoff oder Halogen, eine niedere Alkyl-, niedere Alkoxy-, Aryl-, Aralkyl-, Aralkoxy-, eine Hydroxy-, eine Carboxy-, eine Carboxy-nieder-Alkoxy-, eine Aralkoxycarbonyl-, eine Aralkoxycarbonyl-nieder-Alkoxy-, eine Nitro- oder eine niedere Acylaminogruppe oder jeweils zwei benachbarte Substituenten einen gegebenenfalls durch Halogen substituierten benzoannellierten Rest,

X ein Schwefelatom oder eine gegebenenfalls durch einen niederen Alkyl-, einen Aryl-, einen Aralkyl- oder einen Acylrest substituierte Iminogruppe,

A'' einen Aminosäure- oder einen Peptidrest,

B'' eine in der Peptidchemie übliche oder davon abgeleitete Stickstoffschutzgruppe bedeutet,

eingesetzt werden.

4. Verfahren zur Herstellung von diagnostischen Mitteln gemäß einem der Ansprüche 1 bis 3, dadurch gekennzeichnet, daß man in an sich bekannter Weise einen saugfähigen Träger mit dem üblichen Esterase- und/oder Protease-Substrat, den üblichen zusätzlichen Hilfsstoffen und den Aktivatoren imprägniert.

5. Verfahren gemäß Anspruch 4, dadurch gekennzeichnet, daß die Imprägnierung des saugfähigen Trägers in zwei Schritten erfolgt.

6. Verwendung eines diagnostischen Mittels nach einem der Ansprüche 1 bis 3 zum Nachweis von Leukozyten in Körperflüssigkeiten, insbesondere im Harn.

**Claims**

1. Diagnostic agent for the detection of esterolytic and/or proteolytic enzymes, especially of leukocyte esterases and/or proteases in body fluids, containing a conventional esterase and/or protease substrate, as well as conventional additional adjuvants, characterised in that it additionally contains one or more activators selected from the group of the

a) pyridine derivatives of the general formula I

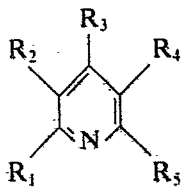

(I)

in which $R_1$, $R_2$, $R_3$, $R_4$ and $R_5$, which can be the same or different, each signify hydrogen or halogen, a lower alkyl or al lower alkoxy group or a vinyl group, which is substituted by an aryl radical optionally carrying one or more lower alkoxy, amino, alkylamino, dialkylamino groups, or by a heterocyclic radical, two adjacent substituents can represent an indeno-or benzo-annellated residue potionally substituted one or more times by halogen, hydroxyl group, lower alkyl or lower alkoxy groups, which in turn can carry a benzo- or pyrido-annellated residue optionally substituted by a lower alkyl group and $R_3$ can additionally stand for a vinyl-quinuclidyl-carbinol group,

b) imidazole derivatives of the general formula II

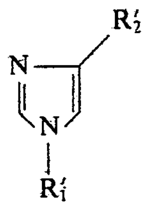

(II)

in which $R_1'$ signifies hydrogen, a lower alkyl group or an aryl radical optinally substituted by a hydroxyl or an acyl group and $R_2'$ hydrogen, an aminoalkyl, N-acylaminoalkyl or a lower aliphatic, optionally unsaturated carboxylic acid residue or a lower aliphatic $\alpha$-amino acid residue optionally acylated on the nitrogen,

c) alcohols of the general formula III

$$X—A—OH \qquad (III)$$

in which X signifies hydrogen or a hydroxyl group and A a hydrocarbon radical,

d) metal complexes of the general formula IV

$$D_m[B(CN)_n(NO)_p] \qquad (IV)$$

in which D signifies an alkali metal ion, B a heavy metal ion, m a whole number from 2 to 5, n a whole number from 4 to 8 and p 0 or 1, the number m being given by the valency of the heavy metal ion and the number n.

2. Diagnostic agent according to claim 1, characterised in that, as additional adjuvants, there are used buffers, complex formers, wetting agents, oxidation agents, film formers, galenical additional materials and/or structure formers.

3. Diagnostic agent according to one of the claims 1 and 2, characterised in that as conventional esterase and/or protease substrate there are used sulphonphthalein esters of the general formula A

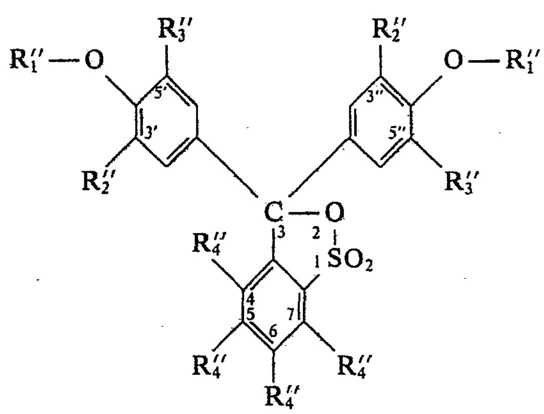

(A)

in which $R_1''$ is a carboxylic acid residue optionally substituted by halogen or a lower alkoxy group, an amino acid or peptide residue provided with a nitrogen protective group conventional in peptide chemistry, $R_2''$ a halogen atom or a lower alkyl group, $R_3''$ and $R_4''$, which can be the same or different,

each a hydrogen or halogen atom; azo dyestuff esters of the general formula B

$$A'—N{=}N—B'(OR)_n \qquad (B)$$

in which A' signifies a five- or six-membered, optionally benzo-annellated residue with one to two heteroatoms from the group N, S, O, which can be optionally substituted one or more times by halogen, lower alkyl and/or lower alkoxy groups or is a phenyl radical substituted one to three times by lower alkyl, lower alkoxy, nitro, sulphonato and/or acylamino groups, B' a benzene, naphthalene or quinoline radical optionally substituted one to two times by sulphonato, lower alkoxy and/or lower alkoxypolyalkyleneoxy groups, R a carboxylic acid residue or an amino acid or peptide residue provided with a nitrogen protective group conventional in peptide chemistry and n the number 1 or 2; or indoxyl esters of the general formula C

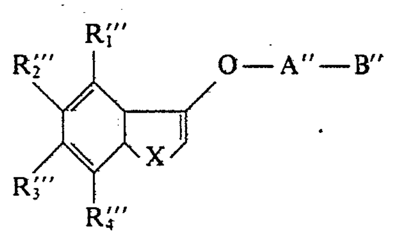

(C)

in which $R_1'''$, $R_2'''$, $R_3'''$, $R_4'''$, which can be the same or different, each signify hydrogen or halogen, a lower alkyl, lower alkoxy, aryl, aralkyl, aralkoxy, a hydroxyl, a carboxy, a carboxy lower alkoxy, an aralkoxycarbonyl, an aralkoxycarbonyl lower alkoxy, a nitro or a lower acylamino group or two adjacent substituents a benzo-annellated residue optionally substituted by halogen, X a sulphur atom or an imino group optionally substituted by a lower alkyl, an aryl, an aralkyl or an acyl radical, A'' an amino acid or peptide residue, B'' a nitrogen protective group conventional in peptide chemistry or derived therefrom.

4. Process for the production of diagnostic agents according to one of claims 1 to 3, characterised in that, in per se known manner, one impregnates an absorbent carrier with the conventional esterase and/or protease substrate, with conventional additional adjuvants and the activators.

5. Process according to claim 4, characterised in that the impregnation of the absorbent carrier takes place in two stages.

6. Use of a diagnostic agent according to one of claims 1 to 3 for the detection of leukocytes in body fluids, especially in urine.

**Revendications**

1. Agent de diagnostic pour la recherche d'enzymes estérolytiques et/ou protéolytiques, en particulier de leucocyte-estérase et/ou de leucocyte-protéase dans des liquides physiologiques, contenant un substrat d'estérase et/ou de protéase habituel ainsi que les agents auxiliaires habituels, caractérisé en ce qu'il contient en outre un ou plusieurs activateurs, choisi parmi les suivants:

a) les dérivés de pyridine de formule générale I

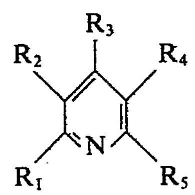

(I)

dans laquelle:

$R_1$, $R_2$, $R_3$, $R_4$ et $R_5$ pouvant être identiques ou différents sont chacun un atome d'hydrogène ou d'halogène, un groupe alkyle inférieur, un groupe alcoxy inférieur ou un groupe vinyle substitué par un reste aryle portant éventuellement un ou plusieurs groupes alcoxy inférieur, amino, alkylamino ou dialkylamino ou par un reste hétérocyclique,

chaque fois deux substituants voisins peuvent représenter un reste indène ou benzénique portant éventuellement comme substituants un ou plusieurs atomes d'halogène, groupes hydroxyle, alkyle inférieur ou alcoxy inférieur et pouvant porter éventuellement encore un autre reste benzénique ou pyridique, éventuellement substitué par un groupe alkyle inférieur,

et $R_3$ peut en outre représenter un groupe vinyl-quinuclidyl-carbinol;

b) les dérivés d'imidazole de formule générale II

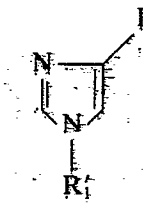

(II)

dans laquelle:

$R_1'$ est de l'hydrogène, un groupe alkyle inférieur ou un reste aryle, substitué éventuellement par un groupe hydroxyle ou acyle, et,

$R_2'$ est de l'hydrogène, un reste amino-alkyle, N-acyl-amino-alkyle ou un reste carboxylique aliphatique inférieur, éventuellement insaturé, ou un reste d'un $\alpha$-amino-acide aliphatique inférieur, éventuellement acylé sur l'atome d'azote;

c) les alcools de formule générale III:

$$X—A—OH \quad (III)$$

dans laquelle:

X est un atome d'hydrogène ou un groupe hydroxyle, et,

A est un reste hydrocarbure;

d) les complexes métalliques de formule générale IV:

$$D_m[B(CN)_n(NO)_p] \quad (IV)$$

dans laquelle:

D est un ion de métal alcalin,

B est un ion de métal lourd,

m est un nombre entier compris entre 2 et 5,

n est un nombre entier compris entre 4 et 8, et

p est égal à 0 ou 1.

le nombre m étant dérivé de la valence de l'ion de métal alcalin et du nombre n.

2. Agent diagnostique selon la revendication 1, caractérisé en ce que l'on emploie, comme agents auxiliaires, des tampons, des formateurs de complexes, des agents mouillants, des agents oxydants, des agents fimogènes, des additifs galéniques et/ou des agents de structuration.

3. Agent de diagnostic selon l'une des revendications 1 et 2, caractérisé en ce que l'on emploie, comme substratshabituels d'estérase et/ou de protéase, des esters du sulfophtaléine, de formule générale A:

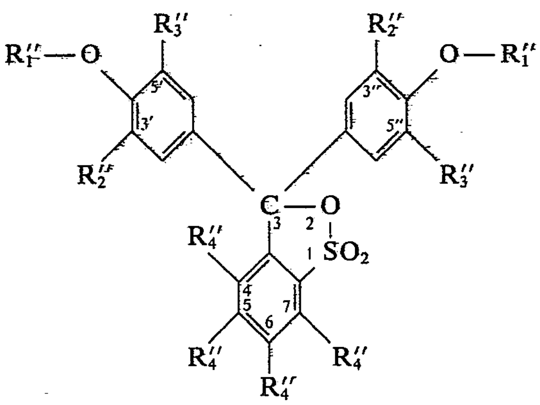

(A)

dans laquelle:

$R_1''$ est un reste carboxylique, éventuellement substitué par un atome d'halogène ou par un groupe alcoxy inférieur, ou un reste d'aminoacide, ou d'un peptide pourvu d'un groupe de protection de l'azote habituellement utilisé dans la chimie des peptides,

$R_2''$ est un atome d'halogène ou un groupe alkyle inférieur,

$R_3''$ et $R_4''$ pouvant être identiques ou différents, sont chacun un atome d'hydrogène ou d'halogène,

des esters de colorants azoïques, de formule générale B:

$$A'—N=N—B'(OR)_n \quad (B)$$

dans laquelle:

A' est un reste à cinq ou six maillons, éventuellement benzéniques, ayant un ou deux hétéro-atomes du groupe N, S, O, éventuellement substitué une ou plusieurs fois par des atomes d'halogène, des groupes alkyle inférieur, et/ou alcoxy inférieur, ou un reste phényle, une à trois fois substitué par des groupes alkyle inférieur, alcoxy inférieur, nitro, sulfonato, et/ou acylamino,

B' est un reste benzène, naphtalène ou quinoline, éventuellement substitué une ou deux fois par des groupes sulfonato, alcoxy inférieur et/ou alcoxy-poly-alcénoxy inférieurs,

R est un reste carboxylique ou un reste d'un amino-acide, ou d'un peptide pourvu d'un groupe de protection de l'azote habituellement utilisé dans la chimie des peptides, et,

n est le nombre 1 ou 2,

ou

des esters d'indoxyle de formule générale C:

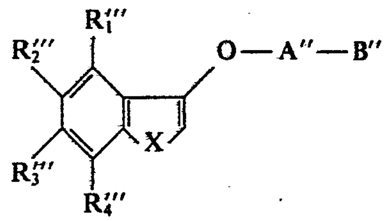

(C)

dans laquelle:

R₁''', R₂''', R₃''', R₄''', pouvant être identiques ou différents, sont chacun un atome d'hydrogène ou d'halogène, un groupe alkyle inférieur, alcoxy inférieur, aryle, aralkyle, aralcoxy, un groupe hydroxyle, carboxy, carboxy-alcoxy inférieur, aralcoxy carbonyle, aralcoxycarbonyle-alcoxy inférieur, nitro ou acylamino inférieur, ou chaque fois deux substituants voisins forment un reste benzénique, éventuellement substitué par de l'halogène,

X est un atome de soufre ou un groupe imino, éventuellement substitué par un reste alkyle inférieur, aryle, aralkyle ou acyle,

A'' est un reste d'un amino-acide ou d'un peptide, et,

B'' est un groupe de protection de l'azote, habituellement utilisé dans la chimie des peptides, ou un dérivé d'un tel groupe.

4. Procédé pour la préparation d'agents de diagnostic selon l'une quelconque des revendications 1 à 3, caractérisé en ce que l'on imprègne, de façon en soi connue, un support absorbant du substrat habituel d'estérase et/ou de protéase, des agents auxiliaires habituels et des activateurs.

5. Procédé selon la revendication 4, caractérisé en ce que l'imprégnation du support absorbant s'effectue en deux étapes.

6. Utilisation d'un agent de diagnostic selon l'une quelconque des revendications 1 à 3, pour la recherche de leucocytes dans les liquides physiologiques, en particulier dans l'urine.